# EUROPEAN PATENT APPLICATION

(11) **EP 4 014 883 A1**
(43) Date of publication of application: **22.06.2022**
(21) Application number: 21160119.0
(22) Date of filing: 02.03.2021
(51) Int. Cl.: A61B 8/00, A61B 8/08, G01S 15/89

(54) **BOUNDARY DETECTION IN ULTRASOUND DATA**

(30) Priority: 18.12.2020 US 202063127630 P
(71) Applicant: Koninklijke Philips N.V., 5656 AG Eindhoven (NL)
(72) Inventor: RAFTER, Patrick Gabriels, 5656 AE Eindhoven (NL); WILD, Sebastian, 5656 AE Eindhoven (NL); PRATER, David, 5656 AE Eindhoven (NL); WAECHTER-STEHLE, Irina, 5656 AE Eindhoven (NL); WEBER, Frank Michael, 5656 AE Eindhoven (NL)
(74) Representative: Philips Intellectual Property & Standards

(57) **Abstract**

A method is provided for determining one or more tissue boundaries within ultrasound image data of anatomical region. The method is based on generating two separate images from the same input ultrasound data, acquired in a single acquisition event. One image is generated with contrast enhancement, so that boundaries surrounding a fluid-containing region are especially well visible and, the second image is generated without this enhancement, or with a different enhancement so that boundaries surrounding tissue regions are especially well visible. An image segmentation procedure is then applied which uses a combination of both images as input and uses both to determine the boundaries within the imaged regions.

## Description

### FIELD OF THE INVENTION

The present invention relates to a method for detecting tissue boundaries within ultrasound data.

### BACKGROUND OF THE INVENTION

Tissue boundary delineation in ultrasound image data is of wide applicability for a range of medical ultrasound applications. In general, it may find use for segmenting boundaries of any organ or other anatomical structure depicted in an ultrasound dataset.

One particular example application for boundary segmentation is within echocardiography.

In particular, despite continued advancements in image quality in echocardiography, the ability to accurately delineate the endocardial borders in an echocardiogram remains a challenge in up to 20% of patients. To aid boundary delineation, a technique known as Left Ventricular Opacification (LVO) can be applied. This is a well-established method for enhancing the endocardial borders in an echocardiogram by means of a suitable contrast agent. Echo contrast agents have been developed and are on the market in most geographies world-wide. Contrast agents greatly enhance the scattered signal from the blood pool, increasing the backscattered signal by orders of magnitude over red blood cells. The contrast agent typically includes microbubbles which enhance echo-reflection.

However, the tissue signal remains extremely strong, even at the harmonic frequencies, limiting the ability to visualize the endocardial borders of the heart. In order to improve the efficacy of ultrasound contrast agents, contrast specific techniques have been developed with the goal to selectively suppress tissue signal. These techniques are known as contrast-enhancement and result in what are termed contrast-enhanced images.

By way of example, these techniques may comprise generating multiple pulses per scan line in a given image and incorporate pulsing schemes which can vary the amplitude, phase or a combination of amplitude and phase from pulse to pulse. The multiple received echoes per scan line are then combined in such a manner to minimize signals from tissue while maintaining signals from the contrast agent microbubbles.

For both cardiac ultrasound images acquired with and without the help of contrast agents, model-based segmentation can be employed to automatically adapt a surface mesh to the cardiac anatomy visible in the image. In a sequence of adaptation steps, the algorithm detects boundaries in the neighborhood of each mesh triangle (using specifically trained boundary detectors) and then adapts the mesh based on these detected image features, and further based on an internal energy derived from prior shape knowledge. This technique works optimally in the event of clearly defined image boundaries at all relevant structures of the cardiac anatomy. A successful segmentation can then be employed for example to derive measurements of ejection fraction without the necessity of further user intervention.

The contrast imaging techniques described above greatly enhance visualization of endocardial borders and are often used in stress echocardiograms to help with detection of wall motion abnormalities. They are also used in measurements of ejection fraction to aid in the evaluation of heart function.

However, to date, there remains a lack of reliable techniques for automating measurement of volumes for images acquired with contrast agents, and hence the ejection fraction is often measured with hand tracing of the corresponding borders.

One important reason why this technical result been difficult to achieve is that the contrast agent in the left ventricular and left atrial cavity obscures the ability to determine where the ventricle ends and the atrium begins. In uncontrasted images, the mitral valve (between the left ventricular and left atrial cavities) can be seen as bright structure. In contrast images however, the blurring of the strong contrast signal obscures the thin mitral valve (which would otherwise in theory be visible as a thin, dark interruption of the bright, contrasted areas). The lack of a clearly defined image feature delineating the boundary between the left ventricle and atrium can significantly hinder the successful application of model-based segmentation.

More generally, this problem of bleeding or blurring of the strong contrast signal can affect visibility in any imaged anatomical region of smaller features located at boundaries of contrast-agent containing regions.

A work-around would be to acquire both a contrast image (with contrast agent administered) and a non-contrast image (without contrast agent). However, this is unsatisfactory for a number of reasons. Firstly, many parameters which one might be seeking to measure using the imaging may not be directly comparable in the two images (e.g. cardiac state, breathing state), and the images may be poorly registered due to shifts in transducer position and orientation). Therefore, a registration step would be required and this can introduce errors or inconsistencies. This in turn would decrease quality of the results. Additionally, the workflow is rendered more complex for the clinician who has to consequently acquire two images with maximally overlapping fields of view.

An improved approach for boundary detection in ultrasound data of anatomical regions would be of value.

### SUMMARY OF THE INVENTION

The invention is defined by the claims.

According to examples in accordance with an aspect of the invention, there is provided a computer-implemented method for determining tissue boundaries within an imaged anatomical region. The method comprises receiving ultrasound data from an anatomical region containing an object of interest which includes one or more tissue boundaries, and wherein the one or more tissue boundaries border a fluid-receiving region. The method further comprises applying a first image-generating procedure to the ultrasound data to generate a first image, the first image-generating procedure adapted to suppress visibility of tissue regions in the generated first image and/or enhance visibility of fluid in the fluid-receiving region in the generated first image. The method further comprises applying a second image-generating procedure, different to the first image-generating procedure, to the ultrasound data to obtain a second image. The method further comprises applying an image segmentation procedure to both the first and second images. The method further comprises determining the one or more tissue boundaries based on the segmentation procedure, or wherein application of the segmentation procedure results in determining of the one or more tissue boundaries. The method further comprises generating a data output representative of the determined one or more tissue boundaries.

The received ultrasound data consists of data for a single data acquisition event or session, e.g. a single scan. A single acquisition event may mean for example data acquired from a single acquisition sequence, meaning a single continuous or uninterrupted sequence of one or more ultrasound pulses and received echoes, or a sequence of acquisition scan lines, where the scan lines may each comprise one or more ultrasound pulses.

The general concept is to perform image segmentation based on two images generated from the same ultrasound data acquired in a single acquisition event. One of the images is generated as a contrast-enhanced image, in which tissue is suppressed and/or fluid is enhanced, and the other is generated without this same tissue suppression or fluid enhancement. The two thus provide different relative contrast representations of the tissue and fluid, the interface between which defines the tissue boundary.

As discussed above, a problem with known contrast-enhanced imaging is that the strong contrast signal of the enhanced visibility regions leads to blurring or outward bleeding at their edges. This can obscure the view of smaller anatomical features, or boundary regions, which are particularly thin such as the mitral valve between the atrial and ventricular chambers of the heart. Thus, by generating two images from the same ultrasound data set, one which is contrast enhanced and one which is not, and performing the segmentation based on both, complementary information is utilized in the segmentation which achieves the benefit of the contrast-enhanced image (i.e. allowing the tissue-fluid interface boundary (e.g. endocardium) to be identified accurately) and while also allowing smaller anatomical structures or thin wall sections (e.g. mitral valve) to be identified.

The two images are generated using the same ultrasound data. There is therefore inherent spatial registration between them.

The image segmentation may be applied separately to the two images, but more preferably comprises a combined segmentation which makes use of both images. In other words, preferably the segmentation is applied simultaneously to both the first and second image.

The first image generating procedure includes image generating steps designed to result in a first image in which visibility of tissue regions is suppressed and/or visibility of fluid in fluid-receiving regions in enhanced compared to an image generated from the ultrasound data and without those same steps, and more particularly compared to the second image.

The first image generating procedure may comprise one or more post-processing steps applied to the received ultrasound data in advance of then generating the image from the post-processed data, wherein the post-processing steps have the effect in the resulting generated first image of suppressing visibility of tissue and/or enhancing visibility of fluid in the fluid-receiving region compared to an image generating procedure which lacks said post-processing steps but which is otherwise the same.

The second image generating procedure is preferably not configured to suppress visibility of tissue regions in the generated second image or to enhance visibility of fluid in the fluid-receiving region in the generated first image. It is thus adapted to result in a second image in which visibility of the tissue regions is greater compared to the first image, and in which the visibility of the fluid is lower compared to the first image.

Optionally, the second image generating procedure may further comprise one or more ultrasound data processing steps for achieving a tissue enhancement and/or fluid suppression effect in the reconstructed image. However this is not essential.

The fluid-receiving region preferably contains a fluid which comprises a contrast agent.

Thus the enhancement of the visibility of fluid in the first image corresponds to enhancement of visibility of contrast-agent in the image. By way of one example, the fluid receiving region may be a heart chamber, and the fluid may be blood.

The contrast agent may comprise microbubbles. These produce strong ultrasound-reflection.

In at least one set of embodiments, the ultrasound data comprises respective ultrasound signals acquired from each of at least three successive ultrasound pulses, two of the three pulses having a first power level, and a third of the three pulses having a second power level different to the first power level. In particular, the received ultrasound data in this example may comprise data corresponding to a plurality of transmit or scan lines. For each transmit line, the data may comprise a set of respective ultrasound signals acquired from each of at least three successive ultrasound pulses.

In general the contrast-agent in the fluid region may have a non-linear response as a function of power of the ultrasound pulses. This is particularly so if the fluid contains a microbubble-based contrast agent. By varying the power level between pulses, and due to the differing power responses of the tissue and contrast agent, this means that the source ultrasound data includes information permitting discrimination between the tissue and fluid regions in the ultrasound data.

In at least one set of examples, the second power level may be double the first power level; and wherein the first image-generating procedure comprises generating the first image based on a combination of the signal from the third pulse with the second power level and at least one of the signals from the two pulses with the first power level.

By generating the first image based on the combination of data obtained with two different power levels, it is possible to selectively enhance the fluid and suppress the tissue.

By using three successive pulses, from the same acquisition period or event, this ensures inherent spatial registration between the ultrasound signals from the three pulses.

In at least one set of examples, the second image-generating procedure comprises generating the second image based on the ultrasound signal from only one of the three pulses. In this way, the second image is in effect a linear image or base image, generated from the ultrasound data from a single one of the pulses, without combination with any other received pulse signals.

In at least one set of examples, the second image-generating procedure may comprise generating the second image based on the ultrasound signal from the third pulse with the second power level. This maximizes power of the third signal, which maximizes overall visibility of the features depicted in the second image.

In at least one set of examples, the first image generating procedure comprises: generating a summed signal by summing the ultrasound signals from the two pulses with the first power level; determining a difference signal based on determining a difference between the summed signal and the signal from the third pulse; and generating the first image based on the difference signal.

Due to the differing power-dependent responses of the tissue and contrast-agent containing fluid, the summed signal will provide a different contrast response than the signal from the third pulse. The difference signal represents this difference in the responses, and thus provides a maximal contrast between the tissue and fluid.

The power level of the different pulses may be configured by adjusting an amplitude of the transmitted ultrasound pulses.

In accordance with one or more embodiments, the object of interest comprises at least a portion of the heart of a subject. The one or more tissue boundaries may in certain examples include the endocardium, and the fluid-containing region includes a ventricular chamber and/or atrial chamber.

In accordance with one or more embodiments, the method may further comprise generating a display output for causing display on a display device of both the first image and the second image, and preferably for further causing display of a representation of the determined one or more tissue boundaries. This aids the clinician using the system in making assessing the heart structure and making a diagnostic conclusion.

Examples in accordance with a further aspect of the invention provide a computer program product comprising code means configured, when run on a processor, to cause the processor to perform a method in accordance with any example or embodiment outlined above or described below, or in accordance with any claim of this application.

Examples in accordance with a further aspect of the invention provide a processing arrangement comprising: an input/output; and at least one processor. The at least one processor is adapted to receive, at the input/output, ultrasound data representative of an anatomical region containing an object of interest which includes one or more tissue boundaries, and wherein the one or more tissue boundaries border a fluid-receiving region. The ultrasound data consists of data for a single acquisition event. The at least one processor is adapted to apply a first image-generating procedure to the ultrasound data to generate a first image, the first image-generating procedure adapted to suppress visibility of tissue regions in the resulting generated first image and/or enhance visibility of fluid in the fluid-receiving region in the generated first image. The processing arrangement is further adapted to apply a second image-generating procedure to the ultrasound data to obtain a second image, the second image-generating procedure different to the first image generating procedure. The processing arrangement is further adapted to apply an image segmentation procedure to both the first and second images. The processing arrangement is further adapted to determine the one or more tissue boundaries based on the segmentation procedure. The processing arrangement is further adapted to generate a data output representative of the determined one or more tissue boundaries.

The fluid-receiving region preferably contains a fluid which comprises a contrast agent. Preferably the method does not comprise administering the contrast agent.

In at least one set of embodiments, the ultrasound data may comprise respective ultrasound signals acquired from each of at least three successive ultrasound pulses, two of the three pulses having a first power level, and a third of the three pulses having a second power level different to the first power level.

In at least one set of examples, the second power level may be double the first power level, and wherein first image-generating procedure comprises generating the first image based on a combination of the signal from the third pulse with the second power level and at least one of the signals from the two pulses with the first power level.

A further aspect of the invention provides an ultrasound system comprising: an ultrasound probe comprising a transducer array for acquiring ultrasound data; a display device; and a processing arrangement in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method according to one or more embodiments;
Fig. 2 shows a workflow of an example embodiment in block diagram form;
Fig. 3 shows an example system according to one or more embodiments; and
Fig. 4 shows an example ultrasound system to further aid understanding of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for determining one or more tissue boundaries within ultrasound data of anatomical region. The method is based on generating two separate images from the same input ultrasound data, acquired in a single acquisition session. One image is generated with contrast enhancement, so that boundaries surrounding a fluid-containing region are especially well visible, and the second image is generated without this enhancement, or with a different enhancement, so that boundaries surrounding tissue regions are especially well visible. An image segmentation procedure is then applied which uses a combination of both images as input and determines the boundaries within the imaged regions.

In other words, embodiments of the present invention propose to improve the determination of relevant anatomical structures by applying segmentation simultaneously to two distinct images generated from the data acquired in a single scan. The first image is constructed with the aim of maximizing the signal from a contrast agent containing region, and for example while suppressing the tissue signal. The second image is derived from the ultrasound data with an aim of providing greater relative visibility of the tissue signal, and in the context of LVO (see discussion above) in particular on the visibility of the mitral valve annulus.

By searching for boundary features in both images during a mesh adaptation process, the complementary information of the two images can be used for an improved segmentation, and thus for more accurate determination of cardiac measurements such as ejection fraction.

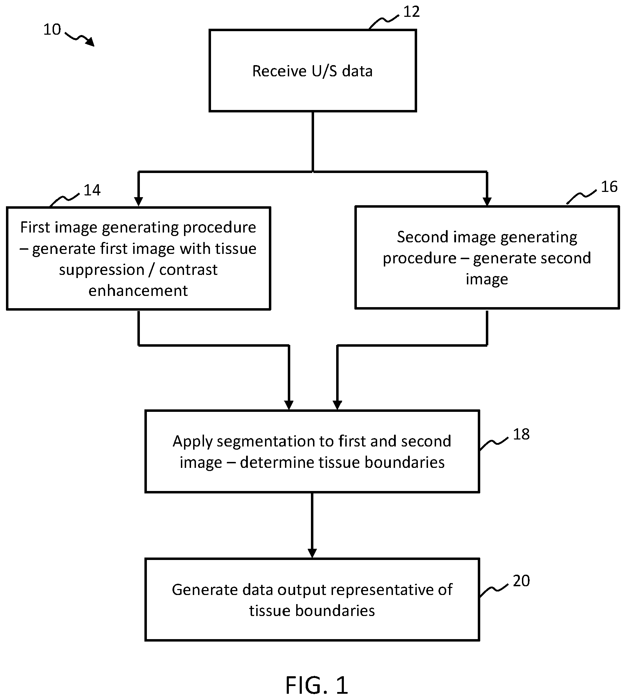

Steps of an example computer implemented method 10 according to one or more embodiments is shown in Fig. 1.

The method 10 comprises receiving 12 ultrasound data from an anatomical region containing an object of interest which includes one or more tissue boundaries, and wherein the tissue boundaries border a fluid receiving region.

The method 10 further comprises applying 14 a first image-generating procedure to the ultrasound data to generate a first image, the first image-generating procedure adapted to suppress visibility of tissue regions in the generated first image and/or enhance visibility of fluid in the fluid-receiving region in the generated first image.

The method 10 further comprises applying 16 a second image-generating procedure, different to the first image-generating procedure, to the ultrasound data to obtain a second image.

The method 10 further comprises applying 18 an image segmentation procedure to both the first and second images. The one or more tissue boundaries are determined based on the segmentation procedure, or the segmentation procedure may result in determination of the one or more tissue boundaries. Preferably, the image segmentation procedure is a combined segmentation which uses both images in combination to arrive the segmentation. As will be explained in more detail later, the image segmentation may automatically adapt a mesh shape based on information derived from both images.

The method 10 further comprises generating 20 a data output representative of the determined one or more tissue boundaries.

The received ultrasound data consists of data for a single acquisition event or session, e.g. a single scan. A single acquisition event may mean for example data acquired from a single acquisition sequence, meaning a single continuous or uninterrupted sequence of one or more ultrasound pulses and received echoes, or a sequence of acquisition scan lines, where the scan lines may each comprise one or more ultrasound pulses.

The fluid-receiving region preferably contains a fluid which comprises a contrast agent. The method may be performed after the contrast agent has been administered. In preferred embodiments, the method does not include administering the contrast agent.

The ultrasound data may be received in real time from an ultrasound imaging apparatus or from an ultrasound transducer unit. Alternatively, it may be received from a datastore on which the data has been recorded in a previous data acquisition session. The ultrasound data may be ultrasound echo data, e.g. RF echo data. Alternatively, it may be data which have been partially post-processed, e.g. beamformed RF data. The data is not yet in the form of a reconstructed image.

The first image generating procedure may comprise a set of post-processing steps applied to the received ultrasound data and a set of image reconstruction steps for then creating an image from the processed data. The post-processing steps have the effect that in the resulting generated (first) image, visibility of the fluid in the fluid-receiving region is enhanced and/or the visibility of the tissue is suppressed compared to an image generated from the received ultrasound data without those post-processing steps. The visibility of these regions is enhanced / suppressed compared to the second image which will be generated by the second image generating procedure.

The second image generating procedure comprises generating a second image from the received ultrasound data. The second image generating procedure may or may not comprise post-processing steps applied to the received ultrasound data in advance of then reconstructing the image. If it does include such steps, these may be configured to have the effect in the resulting (second) image that the tissue regions are enhanced and/or the fluid in the fluid-receiving regions is suppressed compared to image reconstruction performed on the received ultrasound data without these steps, and compared to the first image.

It is noted for avoidance of doubt that although the images are referred to as first and second images, this does not constrain the order in which they are generated. The second image may be generated before the first image for example.

The segmentation procedure will be described in more detail below. It may comprise generating a 3D mesh representative of the locations of the one or more boundaries within the received ultrasound data.

Features in accordance with one set of preferred embodiments will now be outlined.

In accordance with at least this subset of embodiments, a general aim is to generate two images from a set of received ultrasound pulses transmitted and received during a single acquisition process by means of a dual path processing approach, and to use both of them as input for model-based segmentation. The first image, which may be referred to as a contrast-enhanced image in the following, may be based on a typical pulsing scheme used for contrast-agent imaging. It is designed to enhance signals from a contrast agent (e.g. contrast agent microbubbles) but minimize tissue signal. The second image, which may be referred to as a linear image, is constructed with the aim of achieving a strong tissue signal. The use of both images gives rise to complementary information. The linear image can be used to detect boundaries of smaller-dimensioned, e.g. thinner, tissue sections which may otherwise become obscured due to blurring or outward bleeding of the boundaries of the strong contrast-enhanced signal.

By way of example, in the specific context of echocardiography (for example left ventricular opacification (LVO) which was discussed above), the contrast-enhanced (first) image will enable a clear delineation of the endocardial border while the linear image (second image) can be employed for determining the position of the mitral valve annulus which defines the border between the left ventricle and left atrium.

In the segmentation procedure, both images can be used in parallel for detecting boundaries.

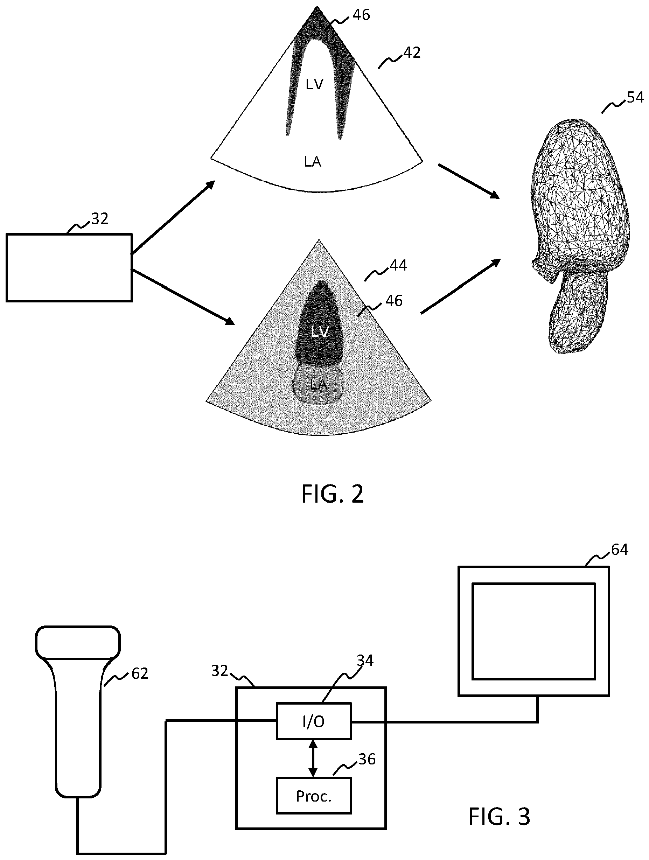

This is schematically illustrated in Fig. 2 which shows a processing arrangement 32 adapted to perform the computer-implemented method in accordance with an embodiment of the invention. The application of the first and second image generating procedures to the received ultrasound data results in generation of the first 42 and second 44 images. Fig. 2 illustrates examples of these two images for received data which is representative of the left ventricle (denoted as LV in the image) and left atrium (LA). The first image is the contrast-enhanced image, and it can be seen that the heart chambers (containing contrast-agent-carrying blood) are brightly visible, while tissue regions 46 are dark. The second image 44 is the linear (second) image, and it can be seen that the tissue 46 is more brightly visible, while the fluid regions appear darker. Grey solid lines schematically indicate a strongly visible delineation between adjacent structures, while dashed grey lines correspond to a weaker delineation. It can be seen that the boundary between the left ventricle (LV) and the left atrium (LA) is not visible at all in the first image 42.

Fig. 2 further shows an example boundary surface mesh 54 generated through a segmentation which is applied to both the first 42 and second 44 images together.

By using both images 42, 44 as input, a segmentation can be achieved which profits from the clear endocardial image features inherent to the contrast agent microbubbles, while still being able to correctly identify the border between the left ventricle and atrium. This facilitates a more accurate automatic derivation of cardiac measurements such as ejection fraction.

One example approach to generating the first image and the second image will now be outlined.

In this approach, it is assumed that the blood of the subject contains a contrast agent, for example a microbubble-containing contrast agent.

In general, contrast imaging techniques are based on the properties of the tissue exhibiting a more linear response to ultrasound waves than microbubbles of a contrast agent. One such technique in contrast imaging is referred to as Power Modulation and takes advantage of the relatively linear response of tissue as the Mechanical Index, or output acoustic amplitude, is changed. Power Modulation consists of three or more transmit pulses (as part of a single transmit sequence) all along the same acoustic path.

In particular, the received ultrasound data in this example comprises data corresponding to a plurality of transmit or scan lines. For each transmit line, the data comprise a set of respective ultrasound signals acquired from each of at least three successive ultrasound pulses. Two of the three pulses have a first power level. A third of the three pulses has a second power level different to the first power level. The power level may be varied by varying an acoustic amplitude of the signals. Although the pulses are labelled as first, second and third, this does not constrain the order in which they are generated. After each ultrasound pulse, a corresponding echo signal is received, so that the received ultrasound data for each transmit line comprises a set of three successive echo signals.

By varying the power level between pulses, and due to the differing power responses of the tissue and fluid, this means that the source ultrasound data includes information permitting discrimination between the tissue and fluid in the ultrasound data.

In at least one set of embodiments, the second power level may be set at double the first power level. It may be approximately double, e.g. double +/- 5%, or +/- 10%. The first image-generating procedure may comprise generating the first image (contrast-enhanced image) based on a combination of the signal from the third pulse with the second power level and at least one of the signals from the two pulses with the first power level.

By generating the first image based on the combination of data obtained with two different power levels, it is possible to selectively enhance the fluid and suppress the tissue. Furthermore, by using three successive pulses, from the same acquisition period or event, this ensures inherent spatial registration between the ultrasound signals from the three pulses.

The first pulse and second pulse are effectively half amplitude, and the third pulse full amplitude. This set of embodiments proposes to construct two distinct images from processing this set of pulses in a dual path approach.

With regards to the first image (the contrast enhanced image), this may be constructed by adding together the first and second pulses of each scan line, and subtracting this result from the third pulse.

In other words, the first image generating procedure may comprise: generating a summed signal for each scan line by summing the ultrasound signals from the two pulses with the first power level; determining a difference signal for each scan line based on determining a difference between the summed signal and the signal from the third pulse, and generating the first image based on the difference signals for the plurality of scan lines.

Since tissue is more linear than the contrast agent, the tissue signal is mostly suppressed leaving only the much more nonlinear contrast agent signal.

With regards to the second image (the linear image) the second image-generating procedure may comprise generating the second image based on the ultrasound signal from only one of the three pulses for each scan line, for example based on the ultrasound signal from the third pulse with the second power level. In other words, the linear image is derived from simply a single pulse (e.g. the third pulse with full amplitude) for each scan line, and thus gives rise to an unsuppressed tissue signal.

As both images are constructed from the same set of pulses for each scan line they are spatially aligned, and thus there is no need for an additional registration step. This is contrast for example to an approach which required generating data in two acquisition sessions, one before contrast agent is administered, and one afterward. Here, due to the delay period between the first and second acquisition sessions, de-registration between the two fields of view would be expected.

Although in the above embodiment, the second image is generated from data of only a single ultrasound pulse, this is not essential. In further variations, the second image generating procedure may include a processing procedure applied to the received ultrasound data for selectively enhancing visibility of the tissue and/or selectively suppressing visibility of the fluid.

Once the first and second images have been generated, the method further comprises applying an image segmentation procedure to both of the images. It is preferably an integrated or combined segmentation procedure in which a single segmentation is derived from a combination of both the first and second images.

By way of example, anatomical model-based segmentation can be used. The skilled person will be aware of a large number of different model-based segmentation algorithms in art.

In some examples, combined application of the segmentation to the both the first and second images may be achieved using already known multi-modal segmentation methods, e.g. those used for a combined analysis of CT and MRI images. One example is outlined for example in the publication: Buerger C., Peters J. et al. (2014) Multi-modal Vertebra Segmentation from MR Dixon for Hybrid Whole-Body PET/MR. In: Yao J., Klinder T., Li S. (eds) Computational Methods and Clinical Applications for Spine Imaging. Lecture Notes in Computational Vision and Biomechanics, vol 17. Springer, Cham.

Details of an example multi-modal segmentation method may also be found in the document WO 2015/189160.

In summary, the segmentation procedure comprises fitting a boundary mesh within the image data based on seeking boundary features and adapting the mesh shape to coincide with the boundary features. When searching boundary features for a given mesh triangle, intensity profiles may be sampled along the normal of the triangle in both the first image and the second image. When a sufficiently strong feature is found in either of the two images, it may be used for attracting the mesh towards that point. Optionally, the relative weight of the boundary features in the two images may be determined, and this used as a further parameter (hyperparameter) within the segmentation procedure. For example.

In accordance with one or more embodiments, where the imaged region is the heart, and in particular the left ventricle and atrium, the method may further comprise determining one or more hemodynamic parameters based on the determined tissue boundaries. For example, cardiac output may be determined based on tracking changes in the left ventricle volume over a single cardiac cycle. Changes in the left ventricle volume can be determined from tracking changes in the outer periphery of the left-ventricle, which can be determined from the segmentation. The received ultrasound data may include data for a series of time points over at least one cardiac cycle. The method as described in this disclosure may be applied recurrently to the data for each of the different time points.

The method further comprises generating a data output representative of the determined one or more tissue boundaries. This may simply be a data package which carries a data representation of the derived boundary, e.g. a set of co-ordinates of triangles of a mesh. Additionally or alternatively, it may be an output for providing a visual representation of the identified one of more boundaries.

For example, in accordance with at least one set of embodiments, the method may comprise generating a display output for causing display on a display device of the determined one or more boundaries. This may be superimposed or overlaid atop one or both of the first and second images for example. Each of the first and second images show different boundary features more clearly: the first showing the interior boundary of the heart chambers more clearly, and the second showing the mitral valve more clearly. By presenting both the first and second images on the display, with the generated boundary mesh superimposed atop each, this allows both of the different types of boundary features to be identified/located by a user within the relevant images.

The display device may be a user interface having a display unit and optionally a user input means.

The user interface may include functionality to permit the user to manually adjust the derived segmentation. For example, in the case of echocardiography, there may be functionality to permit the user to adjust the location of the mitral valve plane which may be visible (mainly) in the linear image with unsuppressed tissue signal.

As discussed above, a preferred application is for segmenting boundaries of cardiac chambers and structures. Hence here, the object of interest represented in the ultrasound data comprises at least a portion of the heart of a subject.

The one or more tissue boundaries may include the endocardium, and the fluid-containing region may include a ventricular chamber and/or atrial chamber.

However, further applications are also possible. In general, any region comprising a fluid-receiving chamber or lumen surrounded by a tissue boundary might be a valuable application area for embodiments of this invention. Examples might include the colon or small intestine, the stomach, or any other fluid-receiving organ.

Examples in accordance with a further aspect of the invention provide a computer program product comprising code means configured, when run on a processor, to cause the processor to perform a method in accordance with in accordance with any example or embodiment outlined above or described below, or in accordance with any claim of this application.

Examples in accordance with a further aspect of the invention provide a processing arrangement comprising: an input/output; and at least one processor adapted to:
receive, at the input/output, ultrasound data representative of an anatomical region containing an object of interest which includes one or more tissue boundaries, and wherein the tissue boundaries border a fluid-receiving region, and wherein the ultrasound data consists of data for a single acquisition event;
apply a first image-generating procedure to the ultrasound data to generate a first image, the first image-generating procedure adapted to suppress visibility of tissue regions in the resulting generated first image and/or enhance visibility of fluid in the fluid-receiving region in the generated first image;
apply a second image-generating procedure to the ultrasound data to obtain a second image, the second image-generating procedure different to the first image generating procedure;
apply an image segmentation procedure to both the first and second images;
determine the one or more tissue boundaries based on the segmentation procedure; and
generate a data output representative of the determined one or more tissue boundaries.

Implementation options and details for each of the features of the above processing arrangement may be understood and interpreted in accordance with the explanations and descriptions provided above in respect of the method aspect of the invention. Any of the examples, options or embodiment features or details described above in respect of the method may be applied or combined or incorporated mutatis mutandis into the present processing arrangement aspect of the invention.

Fig. 3 schematically illustrates components of one example system which may be provided in accordance with a further aspect of the invention. The system comprises a processing arrangement 32 in accordance with any example or embodiment outlined above or described below, or in accordance with any claim of this application. The processing arrangement 32 comprises an input/output 34 and a processor 36 operatively coupled with the input/output. The processing arrangement is arranged to receive ultrasound data from an ultrasound transducer unit 62 which is operatively coupled with the input/output 34. The ultrasound transducer unit may comprise an ultrasound probe for example. The ultrasound transducer unit comprises one or more ultrasound transducers, e.g. a transducer array, for acquiring the ultrasound data. A display device 64 is further provided, operatively coupled with the input/output.

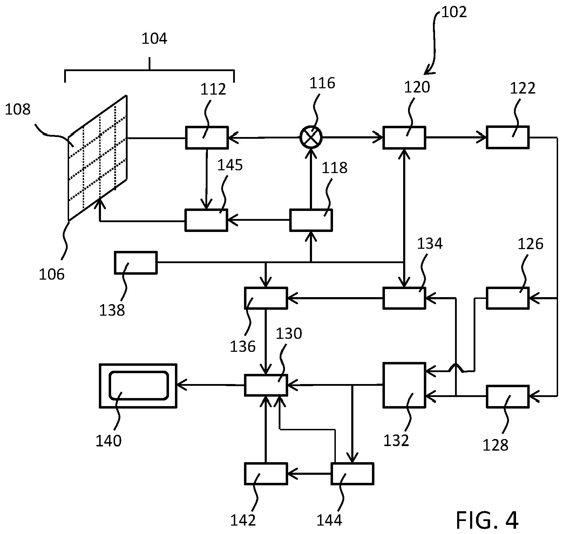

According to any of the above aspects and embodiments of the invention, the ultrasound data may be received in real time from an ultrasound imaging apparatus, or may be received post-acquisition, for example from a datastore on which acquired ultrasound data has been recorded. In the former case, the method may comprise a step of acquiring the ultrasound data. To further aid in understanding of the invention, for example the data acquisition and/or the image reconstruction, the general operation of an exemplary ultrasound system will now be described, with reference to Fig.4.

The system comprises an array transducer probe 104 which has a transducer array 106 for transmitting ultrasound waves and receiving echo information. The transducer array 106 may comprise CMUT transducers; piezoelectric transducers, formed of materials such as PZT or PVDF; or any other suitable transducer technology. In this example, the transducer array 106 is a two-dimensional array of transducers 108 capable of scanning either a 2D plane or a three dimensional volume of a region of interest. In another example, the transducer array may be a 1D array.

The transducer array 106 is coupled to a microbeamformer 112 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is in general entirely optional. Further, the system includes a transmit/receive (T/R) switch 116, which the microbeamformer 112 can be coupled to and which switches the array between transmission and reception modes, and protects the main beamformer 120 from high energy transmit signals in the case where a microbeamformer is not used and the transducer array is operated directly by the main system beamformer. The transmission of ultrasound beams from the transducer array 106 is directed by a transducer controller 118 coupled to the microbeamformer by the T/R switch 116 and a main transmission beamformer (not shown), which can receive input from the user's operation of the user interface or control panel 138. The controller 118 can include transmission circuitry arranged to drive the transducer elements of the array 106 (either directly or via a microbeamformer) during the transmission mode.

The function of the control panel 138 in this example system may be facilitated by an ultrasound controller unit according to an embodiment of the invention.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 118 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 118 can be coupled to control a DC bias control 145 for the transducer array. The DC bias control 145 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 112 and are then passed to a main receive beamformer 120 where the partially beamformed signals from individual patches of transducers are combined into a fully beamformed signal, referred to as radio frequency (RF) data. The beamforming performed at each stage may be carried out as described above, or may include additional functions. For example, the main beamformer 120 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 122. The signal processor 122 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 4 only the receiver beamformers 112, 120 are shown, for simplicity. In the complete system, there will also be a transmission chain with a transmission micro beamformer, and a main transmission beamformer.

The function of the micro beamformer 112 is to provide an initial combination of signals in order to decrease the number of analog signal paths. This is typically performed in the analog domain.

The final beamforming is done in the main beamformer 120 and is typically after digitization.

The transmission and reception channels use the same transducer array 106 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 126 and a Doppler processor 128. The B mode processor 126 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 128 processes temporally distinct signals arising from tissue movement and blood flow for the detection of moving substances, such as the flow of blood cells in the image field. The Doppler processor 128 typically includes a wall filter with parameters set to pass or reject echoes returned from selected types of materials in the body.

The structural and motion signals produced by the B mode and Doppler processors are coupled to a scan converter 132 and a multi-planar reformatter 144. The scan converter 132 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 140. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 142 converts the echo signals of a 3D data set into a projected 3D image as viewed from a given reference point as described in US Pat. 6,530,885 (Entrekin et al.).

The 2D or 3D images are coupled from the scan converter 132, multi-planar reformatter 144, and volume renderer 142 to an image processor 130 for further enhancement, buffering and temporary storage for optional display on an image display 140. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 128 and tissue structure information produced by the B mode processor 126 are coupled to a quantification processor 134. The quantification processor produces measures of different flow conditions such as the volume rate of blood flow in addition to structural measurements such as the sizes of organs and gestational age. The quantification processor may receive input from the user control panel 138, such as the point in the anatomy of an image where a measurement is to be made.

Output data from the quantification processor is coupled to a graphics processor 136 for the reproduction of measurement graphics and values with the image on the display 140, and for audio output from the display device 140. The graphics processor 136 can also generate graphic overlays for display with the ultrasound images. These graphic overlays can contain standard identifying information such as patient name, date and time of the image, imaging parameters, and the like. For these purposes the graphics processor receives input from the user interface 138, such as patient name. The user interface is also coupled to the transmit controller 118 to control the generation of ultrasound signals from the transducer array 106 and hence the images produced by the transducer array and the ultrasound system. The transmit control function of the controller 118 is only one of the functions performed. The controller 118 also takes account of the mode of operation (given by the user) and the corresponding required transmitter configuration and band-pass configuration in the receiver analog to digital converter. The controller 118 can be a state machine with fixed states.

The user interface is also coupled to the multi-planar reformatter 144 for selection and control of the planes of multiple multi-planar reformatted (MPR) images which may be used to perform quantified measures in the image field of the MPR images.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method (10) for determining tissue boundaries within an imaged anatomical region, the method comprising:
receiving (12) ultrasound data from an anatomical region containing an object of interest which includes one or more tissue boundaries, and wherein the one or more tissue boundaries border a fluid-receiving region, and wherein the ultrasound data consists of data for a single acquisition event;
applying (14) a first image-generating procedure to the ultrasound data to generate a first image, the first image-generating procedure adapted to suppress visibility of tissue regions in the resulting generated first image and/or enhance visibility of fluid in the fluid-receiving region in the generated first image;
applying (16) a second image-generating procedure, different to the first image-generating procedure, to the ultrasound data to obtain a second image;
applying (18) an image segmentation procedure to both the first and second images;
determining the one or more tissue boundaries based on the segmentation procedure; and
generating (20) a data output representative of the determined one or more tissue boundaries.

2. A method as claimed in claim 1, wherein the fluid-receiving region contains a fluid which comprises a contrast agent.

3. A method as claimed in claim 2, wherein the ultrasound data comprises respective ultrasound signals acquired from each of at least three successive ultrasound pulses, two of the three pulses having a first power level, and a third of the three pulses having a second power level different to the first power level.

4. A method as claimed in claim 3,
wherein the second power level is double the first power level; and
wherein the first image-generating procedure comprises generating the first image based on a combination of the signal from the third pulse with the second power level and at least one of the signals from the two pulses with the first power level.

5. A method as claimed in claim 4, wherein the second image-generating procedure comprises generating the second image based on the ultrasound signal from only one of the three pulses.

6. A method as claimed in claim 5, wherein the second image-generating procedure comprises generating the second image based on the ultrasound signal from the third pulse with the second power level.

7. A method as claimed in any of claims 4-6, wherein the first image generating procedure comprises:
generating a summed signal by summing the ultrasound signals from the two pulses with the first power level;
determining a difference signal based on determining a difference between the summed signal and the signal from the third pulse; and
generating the first image based on the difference signal.

8. A method as claimed in any of claims 1-7, wherein the object of interest comprises at least a portion of the heart of a subject.

9. A method as claimed in claim 8, wherein the one or more tissue boundaries include the endocardium, and the fluid-containing region includes a ventricular chamber and/or atrial chamber.

10. A method as claimed in any of claims 1-9, further comprising generating a display output for causing display on a display device of both the first image and the second image, and preferably for further causing display of a representation of the determined one or more tissue boundaries.

11. A computer program product comprising code means configured, when run on a processor, to cause the processor to perform a method in accordance with any of claims 1-10.

12. A processing arrangement (32) comprising:
an input/output (34); and
at least one processor (36) adapted to:
receive, at the input/output, ultrasound data representative of an anatomical region containing an object of interest which includes one or more tissue boundaries, and wherein the one or more tissue boundaries border a fluid-receiving region, and wherein the ultrasound data consists of data for a single acquisition event;
apply a first image-generating procedure to the ultrasound data to generate a first image, the first image-generating procedure adapted to suppress visibility of tissue regions in the resulting generated first image and/or enhance visibility of fluid in the fluid-receiving region in the generated first image;
apply a second image-generating procedure to the ultrasound data to obtain a second image, the second image-generating procedure different to the first image generating procedure;
apply an image segmentation procedure to both the first and second images;
determine the one or more tissue boundaries based on the segmentation procedure; and
generate a data output representative of the determined one or more tissue boundaries.

13. A processing arrangement as claimed in claim 12, wherein the fluid-receiving region contains a fluid which comprises a contrast agent.

14. A processing arrangement as claimed in claim 12 or 13, wherein the ultrasound data comprises respective ultrasound signals acquired from each of at least three successive ultrasound pulses, two of the three pulses having a first power level, and a third of the three pulses having a second power level different to the first power level.

15. An ultrasound system comprising:
an ultrasound probe (62) comprising a transducer array for acquiring ultrasound data;
a display device (64); and
the processing arrangement (32) as claimed in any of claims 11 to 14.
